Europäisches Patentamt

European Patent Office

Office européen des brevets

(19)

(11) **EP 1 486 569 B1**

(12) **EUROPEAN PATENT SPECIFICATION**

(45) Date of publication and mention
of the grant of the patent:
**24.05.2006 Bulletin 2006/21**

(51) Int Cl.:
***C12P 7/64*** *(2006.01)*     ***C11C 3/10*** *(2006.01)*

(21) Application number: **02718419.1**

(22) Date of filing: **21.03.2002**

(86) International application number:
**PCT/IB2002/000830**

(87) International publication number:
**WO 2003/080851 (02.10.2003 Gazette 2003/40)**

(54) **METHOD OF TRANSESTERIFICATION OF FAT OR ANALOGUE**

VERFAHREN ZUR UMESTERUNG VON FETT ODER EINEM ANALOGEN

PROCEDE DE TRANSESTERIFICATION DE GRAISSES OU ANALOGUES

(84) Designated Contracting States:
**BE DK GB NL SE**

(43) Date of publication of application:
**15.12.2004 Bulletin 2004/51**

(73) Proprietor: **Fuji Oil Company, Ltd.**
**Osaka-shi,**
**Osaka 542-0086 (JP)**

(72) Inventors:
• **SAGI, Nobuo,**
**Fuji Oil Company, Limited**
**Izumisano-shi,**
**Osaka 598-8540 (JP)**
• **OKADA, Tadayuki,**
**Fuji Oil Company, Limited**
**Izumisano-shi,**
**Osaka 598-8540 (JP)**

• **FUKAZAWA, Masayuki,**
**Fuji Oil Company, Limited**
**Izumisano-shi,**
**Osaka 598-8540 (JP)**

(74) Representative: **Hayes, Adrian Chetwynd et al**
**Boult Wade Tennant,**
**Verulam Gardens**
**70 Gray's Inn Road**
**London WC1X 8BT (GB)**

(56) References cited:
**EP-A- 0 321 777**     **EP-A2- 0 035 883**
**EP-A2- 0 311 045**     **WO-A1-96/10643**
**JP-A- 1 063 385**     **JP-A- 1 096 148**
**JP-A- 1 165 389**     **JP-A- 56 127 094**
**JP-A- 2002 088 392**

**Description**

Technical Field

The present invention relates to an enzymatic method of interesterification of fats and oils.

Background Art

Conventionally, a fat or oil, and a fatty acid or its ester with a monohydric alcohol (a lower alkyl ester of a fatty acid is herein referred to as a "fatty ester") are subjected to enzymatic interesterification to convert triglycerides into a specific triglyceride molecular species and increase its concentration, thereby improving characteristic properties of the fat or oil. In this reaction, when the moisture content in a reaction system is high, the amount of diglycerides which are products of hydrolysis is increased. The diglycerides not only influence the quality of the converted triglyceride which itself is the end product, but also may trigger isomerization of the triglyceride which occurs as a side reaction during the above reaction. In the production of SOS (1,3-distearoyl-2-oleoylglycerol) type hard butter, when the above side reaction occurs, the isomerized triglyceride SSO is formed and further the triglyceride SSS is also formed.

Since these triglycerides have a very great influence on the quality of SOS type hard butter, it is necessary to remove them by means of fractionation, and the like. Although solvent fractionation is an effective means for obtaining a fraction wherein the desired triglyceride is concentrated, such a means requires large-scale facilities, and increases the cost of production. In addition, from the viewpoint of safety of working, preferably, a solvent should not be used. Further, although there is a proposal that diglycerides and/or monoglycerides are removed from a converted triglyceride by hydrolysis with an enzyme specific for them (e.g., JP 62-287 A), this proposal has such a defect that the steps become complicated. Then, it is of importance to suppress the formation of causative diglycerides as much as possible.

A proposal was made to perform interesterification in a reaction system which was dried as much as possible (JP 57-8787 A, etc.). Since then, various proposals have been made to suppress the formation of diglycerides by controlling the moisture content of a reaction system. However, when a free fatty acid is used as a source of an acyl group to be newly introduced into a fat or oil in exchange for that of the fat or oil to decrease the moisture content, the reaction rate of interesterification is lowered too much to desirably decrease the moisture content of a reaction system. As a result, the influence of partial glycerides formed by hydrolysis is still remained. Further, a fatty acid has a high melting point and there is such a problem that the temperature of a reaction system becomes considerably high. Then, the use of a thermostable enzyme is required. Otherwise, an organic solvent is introduced into the system to dissolve the fatty acid and lower the temperature of the system. The introduction of an organic solvent involves considerable investment in facilities and safety requirements.

On the other hand, when a fatty ester is used as a source of an acyl group, a reaction system can be made up at a not so high temperature without introduction of an organic solvent and it is very advantageous from the industrial viewpoint. In this reaction system, the interesterification rate is relatively high and the moisture content of the reaction system therefore can be considerably restricted. However, undesirable triglycerides such as SSS, etc. may be still formed and there is still room for improvement in the quality of a product.

Disclosure of the Invention

An object of the present invention is to provide a method of interesterification in which the desired triglyceride of high quality can be always obtained constantly.

As a result of intensive study of various methods of interesterification, the present inventors have reconsidered the conventional alternative use of an either combination of a fat or oil and a fatty acid, or a fat or oil and a fatty ester, in addition to the restriction of the moisture content of a reaction system, and have found that the formation of diglycerides and SSS triglycerides which is undesirable from the viewpoint of quality can be controlled by using three materials, i.e., a fat or oil, a fatty acid and a fatty ester and adjusting a balance among them. Thus, the present invention has been completed.

That is, the gist of present invention is an enzymatic method of interesterification of fats and oils wherein the fats and oils to be used as a reaction substrate comprises a fat or oil (triglycerides), a fatty acid and a fatty ester, and the acid value of the reaction substrate is 8 to 30.

Best Mode for Carrying Out the Invention

The fat or oil, the fatty acid and the fatty ester to be used as the reaction substrate can be selected from any kinds thereof. Preferably, both acyl groups of the fatty acid and the fatty ester are selected so that they are the desired acyl group to be introduce into the fat or oil and the total amount of the fatty acid and the fatty ester is 0.1 to 20 times, preferably, 0.3 to 10 times as much as the amount of the fat or oil. Typically, in the present invention, the 2-position of the fat or oil to be used in the substrate is unsaturated, the acyl group to be introduced is a saturated fatty acid, and a fat or oil mainly produced is a 1,3-disaturated-2-unsaturated triglyceride.

In the present invention, for converting triglycerides by interesterification of a fat or oil, a free fatty acid and a fatty ester are used together as a source of an acyl group to be newly introduced into the fat or oil in exchange for that of the fat or oil. In this reaction system, triglycerides (TG) and diglyceride (DG), water ($H_2O$) and a free fatty acid (FA) are present in an equilibrium state as shown by the following formula:

$$TG + H_2O = DG + FA$$

wherein, the larger the amount of FA is, the more the hydrolysis reaction toward the right side for producing FA is suppressed. Accordingly, the hydrolysis reaction can be suppressed by adding a free fatty acid to an interesterification reaction system of a fat or oil and a fatty ester, thereby inhibiting the formation of diglycerides and therefore the formation of SSS in the above typical example without lowering the reaction rate and requiring the use of a solvent.

The higher an acid value which represents the amount of an existent free fatty acid is, the more this effect can be enhanced. However, when an acid value exceeds 30, the rate of the main reaction, i.e., interesterification is lowered and the ratio of conversion to the desired triglyceride is also significantly lowered. In addition, crystals of a free fatty acid is liable to be precipitated. For preventing this, it is required to raise the reaction temperature to about 60°C. However, in general, the reaction at such a high temperature is undesirable because inactivation of an enzyme is advanced. Therefore, preferably, the acid value of the reaction substrate is 30 or less, in particular, 20 or less. Further, when the acid value is less than 8, the effect for suppressing the hydrolysis is scarcely recognized. In view of the above, preferably, the acid value is 8 or more, in particular, in case of performing interesterification in a multi-stage fashion, 10 or more.

The smaller the amount of existent water is, the more the reaction toward the right side which consumes water, i.e., the hydrolysis is suppressed. When the amount of water contained in the reaction substrate exceeds 1,800 ppm, the hydrolysis occurs, even if the acid value of the substrate is within the above range. This is undesirable. In order to achieve the object of the present invention, the amount should be 1,800 ppm or less, preferably 100 ppm or less.

An enzyme is reacted with the reaction substrate prepared according to the above conditions to perform the interesterification. Any enzymes originated from microorganisms, plants and animals can be used. Examples of the enzymes include lipases having specificity for 1 and 3 positions of glycerides and derived from microorganisms such as *Rhizopus delemar*, *Mucor miehei*, *Alcaligenes* sp., etc; so-called random type lipases derived from microorganisms such as *Aspergillus niger, Candida cylindracea, Geotricum candidum,* etc.; lipases derived from plants such as soybeans, rice bran, castor seeds, etc.; lipases derived from animal pancreases; and the like. Usually, it is convenient to use commercially available products thereof. As such lipases, in addition to lipases themselves, immobilized lipases, which are obtained by conventional methods such as adsorption method, ion or covalent bond method, inclusion method, and the like, can be used. Further, microorganisms such as mold, yeast, bacteria, etc., which are capable of producing the lipases, may be used as such.

According to the method of the present invention, the production of diglycerides, SSS triglyceride, etc., which is undesirable from the viewpoint of quality, can be suppressed. As a result, the desired triglyceride of high quality can be obtained constantly. Further, when the method of the present invention is performed in a multi-stage fashion, that is, by repeating the following steps 1) to 4), and then distilling off all fatty acids and fatty esters in a reaction mixture without performing the steps 3) and 4) in the final cycle thereof to obtain a remaining fat or oil, it is possible to increase the concentration of the desired triglyceride only by interesterification without performing solvent fractionation:

1) a reaction substrate comprising a fat or oil (triglycerides), a fatty acid and a fatty ester and having an acid value of 8 to 30 is prepared;
2) the reaction substrate is subjected to enzymatic interesterification of fats and oils;
3) all or a part of fatty acids and fatty esters are distilled off a reaction mixture; and
4) fatty acids and fatty esters are added to the remaining fat or oil in the distillation to prepare the reaction substrate as described in the step 1).

Examples

The present invention is further illustrated in detail by the following Examples and Comparative Examples In Examples and Comparative Examples, "parts" and "percents" are by weight.

Example 1

Ethyl stearate (C18, purity 97.8%) and stearic acid (C18, purity 97.2%) were mixed and adjusted to the acid value of 12.5. To this mixed composition of fatty acid/fatty ester (80 parts) was added a deacidified oil of high oleic sunflower oil (acid value 0.13, 20 parts) and the mixture was mixed uniformly. The resultant fat or oil mixture (acid value 10.0) was dehydrated by heating to 110°C under vacuum until the moisture content of the mixture became 30 ppm. After dehydration, molecular sieves 3A was added to the fat or oil mixture (reaction substrate) to further decrease the moisture content to 20 ppm. The resultant reaction substrate was passed through a column packed with a diatomaceous earth carrying a lipase having 1,3-specificity and interesterification activity (immobilized enzyme catalyst, 120 g) at 40°C and a flow rate of 50 g/hr to perform interesterification. The composition of the reaction mixture at the outlet of the column was analyzed by GLC and HPLC at 67 hours after commencement of the reaction.
The results of the analysis are shown in Table 1 together with those of the following Comparative Example 1.

Comparative Example 1

According to the same manner as that described in Example 1, the interesterification was performed except that stearic acid was not used and a deacidified high oleic sunflower oil (acid value 0.13, 20 parts) was added to ethyl stearate (80 parts) to prepare a fat or oil mixture.
The results are shown in Table 1 together with those of the above Example 1.

Table 1

| | Example 1 | | | Comparative Example 1 | | | Comparative Example 2 | |
|------|------|------|------|------|------|------|------|------|
| DG | SOS | SSS | DG | SOS | SSS | DG | SOS | SSS |
| 2.3% | 62.3% | 0.7% | 3.6% | 61.8% | 2.0% | 3.0% | 62.7% | 1.1% |

Comparative Example 2

According to the same manner as that described in Example 1, the interesterification was performed except that the mixed composition of stearic acid/ethyl stearate was adjusted to the acid value of 4.5 (the acid value of the fat or oil mixture was 3.6).
The results are shown in Table 1.

Example 2

Ethyl stearate (C18, purity 92.1%) and stearic acid (C18, purity 97.2%) were mixed and adjusted to the acid value of 15. To this stearic acid/ethyl stearate mixed composition (80 parts) was added a deacidified high oleic sunflower oil (acid value 0.13, 20 parts) to prepare a fat or oil mixture (acid value 12). The mixture was dehydrated by heating under vacuum and molecular sieves 3A was added to adjust the moisture content of 20 ppm. Then, according to the same manner as that of Example 1, interesterification was performed in an column. After the reaction, the mixed composition was subjected to distillation to remove a part of the fatty ester and the fatty acid, thereby concentrating a glyceride oil.
The above fatty ester and fatty acid mixed composition removed by distillation was extremely hydrogenated so that unsaturated fatty esters and unsaturated fatty acids were converted into saturated fatty esters and saturated fatty acid and the resultant was added to the concentrated glyceride oil so as to prepare a mixture having the acid value of 12. Then, the moisture content of the mixture was adjusted to 20 ppm, again and the enzymatic interesterification in the column was performed, again.
The mixed composition obtained after this re-reaction was subjected to distillation to remove all of the fatty esters and fatty acids to obtain an interesterified glyceride oil.
The results of the composition analysis thereof are shown in Table 2 together with those of the following Comparative Example 3.

Comparative Example 3

According to the same manner as that described in Example 2, the interesterification was performed except that the acid value of the stearic acid/ethyl stearate mixed composition was adjusted to 8 (the acid value of the fat or oil mixture was 6.4).

The results are shown in Table 2.

Table 2

| | Example 2 | | | Comparative Example 3 | | | |
|---|---|---|---|---|---|---|---|
| DG | SOS | SSS | SSO | DG | SOS | SSS | SSO |
| 1.9% | 68.3% | 1.2% | 1.7% | 2.5% | 64.5% | 2.7% | 1.6% |

It can be seen from the above description that, in comparison with Comparative Example, the method of interesterification of the present invention suppresses the formation of undesirable by-products by adjusting the amount of the free fatty acid present in a reaction system to a certain level or higher, thereby always obtaining the desired triglycerides of high quality constantly.

**Claims**

1. An enzymatic method of interesterification of fats and oils **characterized in that** the fats and oils to be used as a reaction substrate comprises a fat or oil, a fatty acid and a fatty ester, and the acid value of the reaction substrate is 8 to 30.

2. The enzymatic method of interesterification of fats and oils according to claim 1, wherein the moisture content of the substrate is 1,800 ppm or less.

3. A method of interesterification of fats and oils **characterized in that** the steps of the following 1) to 4) are repeated, and then all fatty acids and fatty esters in a reaction mixture are distilled off without performing the steps 3) and 4) in the final cycle thereof to obtain a remaining fat or oil:

   1) a reaction substrate comprising a fat or oil, a fatty acid and a fatty ester and having an acid value of 8 to 30 is prepared;
   2) the reaction substrate is subjected to enzymatic interesterification of fats and oils;
   3) all or a part of fatty acids and fatty esters are distilled off a reaction mixture; and
   4) fatty acids and fatty esters are added to the remaining fat or oil in the distillation to prepare the reaction substrate as described in the step 1).

**Patentansprüche**

1. Enzymatisches Verfahren zur Zwischenveresterung von Fetten und Ölen, **dadurch gekennzeichnet, dass** die Fette und Öle, die als Reaktionssubstrat verwendet werden, ein Fett oder Öl, eine Fettsäure und einen Fettsäureester umfassen und der Säurewert des Reaktionssubstrats 8 bis 30 ist.

2. Enzymatisches Verfahren zur Zwischenveresterung von Fetten und Ölen gemäss Anspruch 1, worin der Feuchtegehalt des Substrats 1.800 ppm oder weniger beträgt.

3. Verfahren zur Zwischenveresterung von Fetten und Ölen, **dadurch gekennzeichnet, dass** die folgenden Schritte (1) bis (4) wiederholt werden und dann alle Fettsäuren und Fettsäureester in der Reaktionsmischung im Endzyklus ohne Durchführung der Schritte (3) und (4) zum Erhalt eines verbleibenden Fetts oder Öls abdestilliert werden:

   (1) es wird ein Reaktionssubstrat, umfassend ein Fett oder Öl, eine Fettsäure und einen Fettsäureester, und mit einem Säurewert von 8 bis 30, hergestellt;
   (2) das Reaktionssubstrat wird einer enzymatischen Zwischenveresterung von Fetten und Ölen unterworfen;
   (3) alle oder ein Teil der Fettsäuren und Fettsäureester werden aus der Reaktionsmischung abdestilliert; und
   (4) Fettsäuren und Fettsäureester werden zu dem verbleibenden Fett oder Öl bei der Destillation zur Herstellung des Reaktionssubstrats, wie in Schritt (1) beschrieben, zugegeben.

**Revendications**

1. Procédé d'interestérification enzymatique de graisses et d'huiles, **caractérisé en ce que** les graisses et huiles à utiliser comme substrat de réaction comprennent une graisse ou huile, un acide gras et un ester gras, et l'indice d'acide du substrat de réaction est de 8 à 30.

2. Procédé d'interestérification enzymatique de graisses et d'huiles suivant la revendication 1, dans lequel la teneur en humidité du substrat est de 1 800 ppm ou moins.

3. Procédé d'interestérification enzymatique de graisses et d'huiles, **caractérisé en ce que** les étapes 1) à 4) suivantes sont répétées, et ensuite tous les acides gras et les esters gras dans un mélange de réaction sont chassés par distillation sans réaliser les étapes 3) et 4) dans le cycle final de celui-ci pour obtenir une graisse ou huile restante :

   1) un substrat de réaction comprenant une graisse ou huile, un acide gras et un ester gras et ayant un indice d'acide de 8 à 30 est préparé;
   2) le substrat de réaction est soumis à une interestérification enzymatique de graisses et d'huiles;
   3) la totalité ou une partie des acides gras et esters gras est chassée par distillation d'un mélange de réaction; et
   4) des acides gras et des esters gras sont ajoutés à la graisse ou huile restante dans la distillation pour préparer le substrat de réaction tel que décrit dans l'étape 1).